# EUROPÄISCHE PATENTSCHRIFT

(11) **EP 0 551 920 B1**
(45) Veröffentlichungstag und Bekanntmachung des Hinweises auf die Patenterteilung: **13.08.1997**
(21) Anmeldenummer: 93100626.6
(22) Anmeldetag: 15.01.1993
(51) Int. Cl.: A61B 17/32

(54) **Vorrichtung zum Trennen biologischer Strukturen**
Device for cutting biological structures
Dispositif pour couper des structures biologiques

(30) Priorität: 16.01.1992 DE 4200976
(43) Veröffentlichungstag der Anmeldung: 21.07.1993
(73) Patentinhaber: Pein, Andreas, 23627 Gross Grönau (DE)
(72) Erfinder: Pein, Andreas, 23627 Gross Grönau (DE)
(74) Vertreter: Jaap, Reinhard

(56) Entgegenhaltungen:
- EP-A- 0 346 712
- EP-A- 0 411 170
- EP-A- 0 515 983
- DD-A- 298 618
- DE-A- 3 715 418
- DE-A- 4 018 736
- US-A- 2 112 145
- BIOMEDIZINISCHE TECHNIK Bd. 30, Nr. 5, 1985, BERLIN Seiten 99 - 102 KOENIG ET AL. 'Gewebsveränderungen nach Heissluftstrahlkoagulation'

## Beschreibung

Die Erfindung betrifft eine Vorrichtung zum Trennen einer biologischen Struktur, insbesondere des menschlichen Gewebes, bestehend aus einer Druckerzeugungseinrichtung mit einem Druckregelventil für ein Druckmedium, aus einer Einrichtung mit einem Zylinderraum für einen vom Druckmedium belasteten Aufnahmebehälter für ein flüssiges Trennmedium, wobei der auf das Trennmedium ausgeübte Druck ein statischer Druck ist und aus einer manuell handhabbaren Trenneinrichtung, wobei die Druckerzeugungseinrichtung über eine Leitung mit der Einrichtung und der Aufnahmebehälter über eine andere Leitung mit der Trenneinrichtung verbunden sind.

Vorrichtungen dieser Art sind bekannt und werden für die vielfältigsten Trennaufgaben in der Humanchirugie aber auch in der Tierchirugie verwendet. Diese Vorrichtungen, die allgemein auch Jet-Cutting-Systeme genannt werden, werden in der Chirugie zur Trennung biologischer Strukturen der unterschiedlichsten Art verwendet.

Diese Vorrichtungen werden anstelle herkömmlicher chirugischer Trennverfahren immer dann verwendet, wenn eine schonende Therapie erreicht werden soll, d.h. daß wahrend der Trennung unnötige Gewebeverluste weitgehend ausgeschlossen werden sollen, womit das Ziel erreicht wird, die Traumatisierung eines Organs beim chirurgischen Eingriff zu minimalisieren mit dem weiteren Vorteil, daß mit Vorrichtungen dieser Art der Umfang des operativen Eingriffs der Erkrankung angepaßt werden kann, was beispielsweise in der Metastasenchirugie von allerhöchster Bedeutung ist.

Es sind Vorrichtungen bekannt, bei denen das Trennmedium aus einem Vorratsbehälter gefördert und einem Handstück bereitgestellt wird. Derartige Vorrichtungen haben sich nicht bewährt, da das Trennmedium mit allen dazu gehörenden Aggregaten in Berührung kommt und damit die Anforderungen der Sterilisation nicht erfüllt werden können.

Die Sterilhaltung des Trennmediums ist im Prinzip nur bei solchen Vorrichtungen möglich, wo das Trennmedium vom Druckmedium getrennt gehalten wird.

Eine gattungsgemäße Vorrichtung wird in der EP-A 0 411 170 beschrieben. Diese Vorrichtung besitzt eine Druckerzeugungseinrichtung in Form einer Gasdruckflasche, die über ein Druckregelventil mit einer Aufnahmeeinrichtung für ein Softbottle verbunden ist. Der Soft-bottle ist mit einem Trennmedium gefüllt und über eine Leitung mit einem Handstück verbunden.
Das von der Gasdruckflasche bereitgestellte Druckmedium belastet den Soft-bottle an seinem Umfang und verdrängt das sterile Trennmedium zum Handstück, wo es unter Druck austritt.
Diese Vorrichtung hat Nachteile. So wird der Soft-bottle vom Druckmedium umspült und damit allseitig belastet. Durch die Volumenverkleinerung kommt es zu einer schwimmenden Lage des Soft-bottles und zu einem walken der Hülle. Diese Umstände verstärken sich mit Zunahme der Entleerung des Soft-bottles.

Es besteht daher die Aufgabe, eine Vorrichtung der vorliegenden Gattung derart weiterzubilden, daR unter Beibehaltung der Sterilität des Trennmediums stabile und reproduzierbare Druckverhältnisse für das austretende Trennmedium erzielt werden können.

Diese Aufgabe wird durch die kennzeichnenden Merkmale des Anspruchs 1 gelöst.
Die neue Vorrichtung ermöglicht es, in allen Betriebszuständen feinfühlig und stufenlos genaue und reproduzierbare Drücke im Druckmedium zu realisieren. Diese genauen Drücke werden dann auf Grund der bleibenden formschlüssigen Aufnahme der Kartusche im Zylinderraum der Kolben-Zylinder-Einheit über den gesamten Kolbenhub ohne Abweichungen auf das Trennmedium übertragen und sorgen dafür, daR sich der Trennmediumstrom stets, auch nach dem Austritt aus der Düse des Handstückes, im bevorzugten laminaren Strömungsbereich bewegt. Diese kavitationsfreie und laminare Strömung ist letztlich maßgebend für die Ausbildung eines scharfkantigen Wasserstrahles ohne Vernebelungen und Verwirbelungen, der auch nach dem Austritt aus der Düse über eine relativ weite Entfernung erhalten bleibt.
Das ermöglicht eine noch schonendere Trennung des Gewebes. Außerdem wird zusätzlich für die den operativen Eingriff vornehmende Person eine gute Sicht auf das Operationsfeld geboten.
Die Anforderungen an die Sterilität der Trennflüssigkeit werden auf einfache Weise erfüllt, da das Trennmedium stets separat gehalten wird und mit keinerlei Elementen der Druckerzeugungseinrichtung in Berührung kommt.

Bei einer zweckdienlichen Ausgestaltung der Vorrichtung wird eine Vielfachverbindung eingesetzt, um mehrere Handstücke gleichzeitig einsetzen zu können, so daß beispielsweise während eines Operationsvorganges unterschiedliche Handstücke für den jeweiligen operativen Schritt wechselweise ausgewählt werden können.

Bei einer weiteren Ausgestaltung der Vorrichtung wirkt mit dem Handstück eine Kühleinrichtung zusammen, mit der eine gezielte Änderung des Aggregatzustandes des aus der Trenneinrichtung austretenden Trennmediums ausführbar ist und zwar derart, daß infolge der Änderung des Aggregatzustandes das Trennmediums neben weiterhin flüssigen Bestandteilen auch kristallförmiges Trennmedium enthält. Eine derartige Ausgestaltung wird vorzugsweise immer dann gewählt, wenn im Mund-, Kiefer- und Gesichtsbereich knochenchirurgische Eingriffe vorgenommen werden müssen, wobei ein auf vorangehend beschriebene Weise zusammengesetztes Trennmedium schonend auch zur Trennung von Knochen geeignet ist.

Eine andere Ausgestaltung der Vorrichtung zeigt das Zusammenwirken des Handstückes mit einer Heizeinrichtung. Mit der gezielten Erwärmung des Trennmediums wird ein sehr wirksames Koagulationsinstrument zum Verschluß der durchtrennten Gefäße geschaffen. Auch kann damit die Elastizität des zu trennenden Gewebes verändert werden, wodurch ein besseres und schonenderes Trennergebnis erreicht wird.

Grundsätzlich ist die Verwendung von Laserstrahlen in der Chirurgie seit langem bekannt. Mit Lasern werden beispielsweise Nierensteine zertrümmert, wobei sich aber herausgestellt hat, daß die Applikation des Lasers auf den zuzerstörenden Nierenstein selbst Schwierigkeiten bereitet, da überwiegend in Laserstrahlachsrichtung bei der Applikation des Laserstrahls auf den Nierenstein den Nierenstein zerstörende Kraftkomponenten entstehen und auf ihn einwirken. Dieses hat zur Folge, daR der Nierenstein selbst nicht in eine möglichst große Vielzahl von Einzelteilen zerlegt wird, sondern nach einmaliger Applikation weiterhin große Bruchstücke vorliegen. Um zu erreichen, daß der Nierenstein, wie eigentlich angestrebt, bei der Applikation eines Laserstrahls in eine Vielzahl möglichst kleiner Einzelbestandteile zerfällt, ist es vorteilhaft, daß bei der Vorrichtung im Bereich des Handstückes ein Laserstrahl in das Trennmedium eingekoppelt wird, wobei der Laserstrahl und das Trennmedium in im wesentlichen gleicher Richtung aus dem Handstück austreten. Eine derartige Kombination aus unter hohem Druck befindlichem strahlförmigen Trennmedium und dazu im wesentlichen achsparallel austretendem Laserstrahl schafft ein hervorragendes Mittel, um beispielsweise einen Nierenstein in der gewünschten Art zu zerstören, da durch die auf den Nierenstein ausgeübte Wirkung des Trennmediums quasi ein Loch geschaffen wird, in das der Laserstrahl eintritt, so daß der Laserstrahl dann auch im wesentlichen zu seiner Austrittsachse radiale Kraftkomponeneten im Nierenstein induzieren kann, so daß tatsächlich eine Zertrümmerung in viele Bestandteile die Folge ist.

Die Erfindung wird nun unter Bezugnahme auf die einzige nachfolgende schematische Zeichnung anhand eines Ausführungsbeispieles beschrieben, Diese zeigt;
in Form eines erweiterten Blockschaltbildes die Vorrichtung mit ihren Einzelkomponenten. wobei vier unterschiedlich ausgestaltete Trenneinrichtungen gleichzeitig an die Vorrichtung angeschlossen sind.

Die Vorrichtung 10 besteht druckerzeugungsseitig im wesentlichen aus einer Druckerzeugungseinrichtung 12 und einer Kolben-Zylinder-Einrichtung 15. Die Druckerzeugungseinrichtung 12 umfaßt eine Pumpeneinrichtung zur Erzeugung hydraulischen oder pneumatischen Drucks, der auf die Kolbenseite der Kolben-Zylinder-Einrichtung 15 wirkt. Die Druckerzeugungseinrichtung 12 ist dazu über eine Leitung 23 mit der Kolben-Zylinder-Einrichtung 15 verbunden, wobei in der Leitung 23 das Druckmedium 16, beispielsweise Hydrauliköl oder -luft, gefördert wird. Zur Aufrechterhaltung eines konstanten Drucks im Kolbenraum 24 der Kolben-Zylinder-Einrichtung 15 sind in die Leitung 23 auf bekannte Weise ein Druckregelventil 25, ein Rückschlagventil 26, ein Drucksensor 27 und ein Wegeventil 28 geschaltet, wobei über den Drucksensor 27 das Druckregelventil 25 zur Steuerung im wesentlichen konstanten, vorbestimmten Drucks des Druckmediums 16 gesteuert wird. Das Wegeventil 28 dient dazu, den Kolben der Kolben-Zylinder-Einrichtung 15 in seiner Bewegung umkehrbar zu gestalten, wenn nach Ausführung eines vorbestimmten Arbeitshubes des Kolbens der Kolben wieder in seiner Ausganglage zurückgeführt werden muß, was im einzelnen noch weiter unten beschrieben wird.

Getrennt vom Hydraulik- bzw. Pneumatikkreis des Druckmediums 16 ist der Zylinderraum 17 der Kolben-Zylinder-Einrichtung 15 angeordnet. Im Zylinderraum 17 wird das eigentliche flüssige Trennmedium 11, das in der Regel aus Wasser besteht, aufgenommen. Um eine hohe Sterilität des trennmediumseitigen Teils der Vorrichtung 10 zu gewährleisten, ist das Trennmedium 11 in einer Kartusche aufgenommen, die ihrerseits wiederum formschlüssig im Zylinderraum 17 der Kolben-Zylinder-Einrichtung 15 aufgenommen wird.

Der Zylinderraum 17 ist über eine Leitung 14 für das Trennmedium 11 mit einer Ventileinrichtung 18 verbunden, mittels der der Fluß des Trennmediums 11 in der Leitung 14 steuerbar und/oder regelbar ist, wobei über diese Ventileinrichtung 18 auch der Austritt des Trennmediums 11 aus der manuell handhabbaren Trenneinrichtung 13 (Handstück) über einen hier nicht gesondert dargestellten Fußschalter oder ein beliebiges anderes geeignetes Auslöseorgan ausgelöst wird. Die Ventileinrichtung 18 kann auch dazu dienen, mittels hier nicht gesondert dargestellter elektronischer Einrichtungen derart gesteuert zu werden, daß das Trennmedium 11 in gepulster Form aus der Trenneinrichtung 13 austritt.

Der Ventileinrichtung nachfolgend ist eine Vielfachverbindung 29 angeordnet, an die wahlweise eine Mehrzahl von Trenneinrichtungen 13 anschließbar sind. Die Leitung 14 (140, 141, 142, 143) führt in dem in der Fig. dargestellten Ausführungsbeispiel zu 4 Trenneinrichtungen 13, die in der figürlichen Darstellung unterschiedlich ausgebildet sind. Die über die Leitung 143 zur Trenneinrichtung 13 führende Ausgestaltung stellt gewissermaßen die Standardausgestaltung als Teil der Vorrichtung 10 dar. Aus der schon erwähnten manuell betätigbaren Trenneinrichtung 13 tritt bei entsprechender Betätigung der Ventileinrichtung 18 und ggf. einer zweiten, nicht gesondert dargestellten, an der Trenneinrichtung 13 ausgebildeten Auslöseeinrichtung das Trennmedium 11 aus, und zwar aufgrund des von der Kolben-Zylinder-Einrichtung 15 auf das im Zylinderraum 17 angeordnete Trennmedium 11 ausgeübten Drucks, der ein statischer Druck ist.

Die über die Leitung 142 verbundene Trenneinrichtung 13 ist gegenüber der vorbeschriebenen Standardform mit einer in der Leitung 142 angeordneten Heizeinrichtung 20 versehen. Über die Heizeinrichtung 20 kann die Temperatur des aus der Trenneinrichtung 13 austretenden Trennmediums 11 gesteuert und/oder geregelt werden, wobei beispielsweise die Temperatur im Bereich von 45 bis 80° C steuerbar und/oder regelbar ist. Durch diese Maßnahme wird ein einfaches Koagulationsinstrument zum Verschluß der Gefäße während des Trennvorganges geschaffen.

Die über die Leitung 141 mit der Ventileinrichtung 18 verbundene Trenneinrichtung 13 wirkt mit einer Kühleinrichtung 19 zusammen, mit der eine gezielte Änderung des Aggregatszustandes des aus der Trenneinrichtung 13 austretenden Trennmediums 11 ausführbar ist, d.h. es sind gezielt Eiskristalle im Trennmedium 11 erzeugbar, das dann als Gemisch zusammen mit flüssigen Bestandteilen des Trennmediums 11 aus der Düse der Trenneinrichtung 13 austritt, wobei diese Ausgestaltung insbesondere für knochenchirurgische Trennungen im Mund-, Kiefer- und Gesichtsbereich Anwendung finden können.

Die über die Leitung 140 mit der Ventileinrichtung 18 verbundene Trenneinrichtung 13 ist derart gestaltet, daß ein von einer hier nicht gesondert dargestellten Laserquelle zugeführter Laserstrahlen in das Trennmedium 11 einkoppelbar ist. Der Laserstrahl 21 und das Trennmedium 11 treten dann entsprechend der Richtung des Pfeiles in im wesentlichen gleicher Richtung aus der Trenneinrichtung 13 heraus, d.h. der Laserstrahl 21 wird dann nach dem Austritt aus der Trenneinrichtung 13 im Trennmedium 11 geführt.

Bei der Inbetriebnahme und Funktion der Vorrichtung 10 wird zunächst mittels der Druckerzeugungseinrichtung 12 ein auf den Kolbenraum 24 der Kolben-Zylinder-Einrichtung 15 einwirkender konstanter Druck hergestellt. In dem Zylinderraum 17 ist zuvor das Trennmedium 11 auf oben beschriebene Weise eingeführt worden. Über die Ventileinrichtung 18 wird mittels der erwähnten, hier nicht gesondert dargestellten elektronischen Einrichtungen, ggf. rechnergestützt, der Arbeitsdruck des Trennmediums 11 mit einer Reproduktionsgenauigkeit von 0,03 % geregelt, wobei der Arbeitsdruck des Trennmediums 11 bis 500 bar betragen kann, und der durchschnittliche normale Arbeitsdruck des Trennmediums 11 im Bereich bis zu 100 bar liegt. Unter diesem Druck tritt dann das Trennmedium 11 aus der Trenneinrichtung 13 über eine hier nicht gesondert dargestellte Düse aus, die in der Trenneinrichtung 13 angeordnet ist, wobei der Düsendurchmesser beispielsweise 50 µm beträgt. Während eines operativen Eingriffs mit der Vorrichtung 10 können auch eine Mehrzahl von Trenneinrichtungen 11 gleichzeitig mit dem Trennmedium 11 versorgt werden, so daß der Operateur für die verschiedesten Operationsschritte unterschiedlich ausgestaltete Trenneinrichtungen 13, wie sie beispielsweise in der Fig. dargestellt sind, zur Verfügung hat.

### Bezugszeichenliste

- 10: Vorrichtung
- 11: Trennmedium
- 12: Druckerzeugungseinrichtung
- 13: Trenneinrichtung
- 14: Leitung (für Trennmedium)
- 15: Kolben-Zylinder-Einrichtung
- 16: Druckmedium
- 17: Zylinderraum
- 18: Ventileinrichtung
- 19: Kühleinrichtung
- 20: Heizeinrichtung
- 21: Laserstrahl
- 22: Austrittsrichtung
- 23: Leitung
- 24: Kolbenraum
- 25: Druckregelventil
- 26: Rückschlagventil
- 27: Drucksensor
- 28: Wegeventil
- 29: Vielfachverbindung

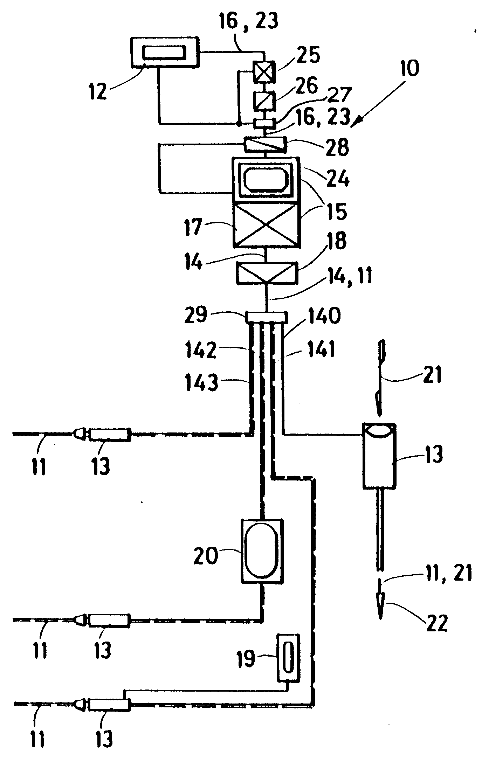

## Patentansprüche

1. Vorrichtung (10) zum Trennen biologischer Strukturen, insbesondere des menschlichen Gewebes, bestehend
- aus einer Druckerzeugungseinrichtung (12) mit einem Druckregelventil (25) für ein Druckmedium (16),
- aus einer Einrichtung mit einem Zylinderraum für einen vom Druckmedium belasteten Aufnahmebehälter für ein flüssiges Trennmedium (11), wobei der auf das Trennmedium ausgeübte Druck ein statischer Druck ist und
- aus einer manuell handhabbaren Trenneinrichtung (13),
- wobei die Druckerzeugungseinrichtung (12) über eine Leitung mit der Einrichtung und der Aufnahmebehälter über eine andere Leitung (14) mit der Trenneinrichtung (13) verbunden sind,
**dadurch gekennzeichnet**, daß die Einrichtung aus einer Kolben-Zylinder-Einrichtung (15) besteht, deren Kolben vom geregelten Druckmedium (16) belastet wird und dessen Bewegung über ein wegeventil (28) umkehrbar ist und deren Zylinderraum (17) den Aufnahmebehälter für das Trennmedium aufnimmt, wobei der Aufnahmebehälter aus einer formschlüssigen Kartusche besteht.

2. Vorrichtung nach Anspruch 1,
**dadurch gekennzeichnet**, daß über eine Vielfachverbindung (29) eine Mehrzahl von Trenneinrichtungen (13) gleichzeitig anschließbar und/oder betreibbar sind.

3. Vorrichtung nach Anspruch 2,
**dadurch gekennzeichnet**, daß mit der handhabbaren Trenneinrichtung (13) eine Kühleinrichtung (19) zusammenwirkt, mit der eine gezielte Änderung des Aggregatzustandes des aus der Trenneinrichtung (13) austretenden Trennmediums (11) ausführbar ist.

4. Vorrichtung nach Anspruch 2,
**dadurch gekennzeichnet**, daß mit der handhabbaren Trenneinrichtung (13) eine Heizeinrichtung (20) zusammenwirkt, mit der die Temperatur des aus der Trenneinrichtung (13) austretenden Trennmediums (11) steuerbar und/oder regelbar ist.

5. Vorrichtung nach Anspruch 2,
**dadurch gekennzeichnet**, daß im Bereich der handhabbaren Trenneinrichtung (13) ein Laserstrahl (21) in das Trennmedium (11) einkoppelbar ist, wobei der Laserstrahl (21) und das Trennmedium (11) in im wesentlichen gleicher Richtung (22) aus der Trenneinrichtung (13) austreten.

## Claims

1. Apparatus (10) for separating biological structures, in particular of human tissue, consisting
- of a pressure generating device (12) with a pressure regulating valve (25) for a pressure medium (16),
- of a device with a cylinder chamber for a receptacle, which is loaded by the pressure medium, for a liquid separation medium (11), wherein the pressure exerted on the separation medium is a static pressure and
- of a separating device (13) which can be manually manipulated,
- wherein the pressure generating device (12) is connected via one line to the device and the receptacle is connected via another line (14) to the separating device (13),
characterised in that the device consists of a piston-cylinder device (15), the piston of which is loaded by the regulated pressure medium (16) and the movement of which can be reversed by a directional control valve (28) and the cylinder chamber (17) of which accommodates the receptacle for the separation medium, wherein the receptacle consists of a form-fitting cartridge.

2. Apparatus according to claim 1, characterised in that a plurality of separating devices (13) can be simultaneously connected and/or operated via a multiple connection (29).

3. Apparatus according to claim 2, characterised in that a cooling device (19) cooperates with the manipulatable separating device (13), by means of which cooling device a specific change in the state of aggregation of the separation medium (11) emerging from the separating device (13) can be effected.

4. Apparatus according to claim 2, characterised in that a heating device (20) co-operates with the manipulatable separating device (13), by means of which heating device the temperature of the separation medium (11) emerging from the separating device (13) can be controlled and/or regulated.

5. Apparatus according to claim 2, characterised in that a laser beam (21) can be coupled into the separation medium (11) in the region of the manipulatable separating device (13), wherein the laser beam (21) and the separation medium (11) emerge from the separating device (13) in essentially the same direction (22).

## Revendications

1. Dispositif (10) de séparation de structures biologiques, notamment de tissu humain, constitué
• d'un dispositif de production de pression (12) avec une vanne de régulation de pression (25) pour un milieu de pression (16),
• d'un dispositif avec une chambre cylindrique pour un récipient de réception chargé par le milieu de pression pour un milieu de séparation liquide (11) où la pression exercée sur le milieu de séparation est une pression statique et,
• d'un dispositif de séparation (13) fonctionnant manuellement,
• où le dispositif de production de pression (12) est relié par une conduite au dispositif et le récipient de réception est relié par une autre conduite (14) au dispositif de séparation (13),
caractérisé en ce que
le dispositif est constitué d'un dispositif piston-cylindre (15) dont le piston est mis en Charge par le milieu de pression (16) régulé et dont le déplacement peut être inversé par un distributeur (28) et dont la chambre cylindrique (17) reçoit le récipient de réception du milieu de séparation, où le récipient est constitué d'une cartouche à emboîtement de forme,

2. Dispositif selon la revendication 1,
caractérisé en ce que
par l'intermédiaire d'une connexion multiple (29), on peut brancher et/ou actionner simultanément une pluralité de dispositifs de séparation (13).

3. Dispositif selon la revendication 2,
caractérisé en ce qu'
un dispositif de refroidissement (19) coopère avec le dispositif de séparation (13) actionnable à la main, grâce auquel on peut réaliser une modification appropriée de l'état d'agrégat du milieu de séparation (11) sortant du dispositif de séparation (13).

4. Dispositif selon la revendication 2,
caractérisé en ce qu'
un dispositif de réchauffement (20) coopère avec le dispositif de séparation (13) actionnable à la main, grâce auquel on peut commander et/ou réguler la température du milieu de Séparation (11) sortant du dispositif de séparation (13).

5. Dispositif selon la revendication 2,
caractérisé en ce que
dans le domaine du dispositif de séparation (13) actionnable à la main, an peut introduire un faisceau laser (21) dans le milieu de séparation (11), où le faisceau laser (21) et le milieu de séparation (11) sortent essentiellement dans le même sens (22) du dispositif de séparation (13).
